# EUROPEAN PATENT SPECIFICATION

(11) **EP 3 636 254 B1**
(45) Date of publication and mention of the grant of the patent: **24.02.2021**
(21) Application number: 19202258.0
(22) Date of filing: 09.10.2019
(51) Int. Cl.: A61K 31/132, A61K 31/455, A61K 31/7016, A61P 39/06

(54) **ANTIOXIDANT COMPOSITIONS**
ANTIOXIDATIVE ZUSAMMENSETZUNGEN
COMPOSITIONS ANTIOXYDANTES

(30) Priority: 10.10.2018 IT 201800009318
(43) Date of publication of application: 15.04.2020
(73) Proprietor: Frati, Luigi, 00136 Roma (RM) (IT); Carnevale, Roberto, 04022 Fondi (Latina) (IT); Sciarretta, Sebastiano, 00136 Roma (IT); Frati, Giacomo, 00136 Roma (IT); Valenti, Valentina, 00136 Roma (IT); Marullo, Antonino, 00135 Roma (IT)
(72) Inventor: Frati, Luigi, 00136 Roma (RM) (IT); Carnevale, Roberto, 04022 Fondi (Latina) (IT); Sciarretta, Sebastiano, 00136 Roma (IT); Frati, Giacomo, 00136 Roma (IT); Valenti, Valentina, 00136 Roma (IT); Marullo, Antonino, 00135 Roma (IT)
(74) Representative: Cesa, Roberta

(56) References cited:
- CN-A- 108 245 513
- US-A1- 2012 178 755
- RYOSUKE ECHIGO ET AL: "Trehalose treatment suppresses inflammation, oxidative stress, and vasospasm induced by experimental subarachnoid hemorrhage", JOURNAL OF TRANSLATIONAL MEDICINE, BIOMED CENTRAL, vol. 10, no. 1, 30 April 2012 (2012-04-30) , page 80, XP021133143, ISSN: 1479-5876, DOI: 10.1186/1479-5876-10-80
- B. J. DEBOSCH ET AL: "Trehalose inhibits solute carrier 2A (SLC2A) proteins to induce autophagy and prevent hepatic steatosis", SCIENCE SIGNALING, vol. 9, no. 416, 23 February 2016 (2016-02-23), page ra21, XP055406225, US ISSN: 1945-0877, DOI: 10.1126/scisignal.aac5472
- ZHANG MING ET AL: "Synthesis and Determination of Absolute Configuration of Lentztrehalose A.", CHEMICAL & PHARMACEUTICAL BULLETIN 2015, vol. 63, no. 11, 2015, pages 961-966, XP002791886, ISSN: 1347-5223

## Description

### Field of invention

The present description refers to antioxidant compositions particularly suitable for contrasting inflammatory processes.

### Invention background

Oxidative stress, an expression coined for the first time by Sies in 1985, defines a transient or permanent disturbance between the cellular production of reactive oxygen species (ROS) and the availability of antioxidant species (Sies, 2015). This disturbance - known for contributing to the pathogenesis of various diseases (Rahman et al., 2012) - involves the onset of both cellular lesions due to a direct oxidation of cellular proteins, lipids and DNA and alterations in signalling pathways that modify the cellular response favouring, for example, the death of the cells (Birben et al., 2012).

Several factors contribute to the genesis of oxidative stress. Xenobiotics, such as radiation, drugs and habits, such as, for example, smoking as well as environmental agents interact with the cellular sources of ROS, inducing their production. Mitochondria, endoplasmic reticulum, peroxisomes and enzyme systems, such as NADPH oxidase, NADP and xanthine oxidase, are some of the main intracellular sources of ROS (Pizzino et al., 2017) . When produced in excess, ROS - which include different reactive species such as, for example, superoxide (-O₂), hydroxyl radicals (HO), hydrogen peroxide (H₂O₂) - are involved in various diseases, such as cancer, neurodegenerative diseases, diabetes and cardiovascular diseases, since they promote the inflammatory process.

The inflammatory process is commonly considered a complex reaction in the vascularized connective tissue in response to exogenous and endogenous stimuli. The ultimate goal of this response is to rid the body of the initial cause of the cell injury and the consequences of that injury. However, a prolonged or unregulated inflammatory process can cause tissue damage and is the cause of many chronic diseases. Several studies support an interdependent relationship between inflammation and oxidative stress and their involvement in various chronic diseases including diabetes and diabetic complications, hypertension and cardiovascular diseases, neurodegenerative diseases, alcoholic liver disease, chronic kidney disease, cancer and aging (Biswas, 2015). Indeed, under pathological inflammatory conditions, ROS over-production by phagocytic cells can occur, which can therefore induce localized oxidative stress and tissue lesions (Fialkow et al., 2007).

In addition to oxidative stress, a complex association has also been identified between inflammation and autophagy.

Autophagy is an intracellular cytoprotective process that mediates protein degradation, organelle turnover, recycling of cytoplasmic components in conditions of nutrient deprivation and cellular stress (Levine et al., 2004). Furthermore, autophagy plays an important role in the removal of excess cellular ROS allowing the maintenance of a redox balance (Li et al., 2015) . The degraded materials in the autophagosome are then used for anabolic reactions, to support energy demands and to provide simple molecules deriving from the degradation process that can be reused by cells for other functions. Autophagy, therefore, helps cells adapt to energy and stress changes by supporting cellular metabolism, homeostasis and survival (Mizushima, 2007). Insufficient autophagic activity can contribute to cell death. Numerous studies have shown that inhibition of autophagic flow can contribute to the pathogenesis of cardiovascular diseases, diabetes, inflammatory disorders, cancer and physical stress (Levine et al., 2008). Furthermore, autophagy influences the development, homeostasis and survival of inflammatory cells, including macrophages, neutrophils and lymphocytes, which play critical roles in the development and pathogenesis of inflammation (Qian et al., 2017).

Considering that oxidative stress and an autophagic deficiency associated with inflammation are common in many diseases, the possibility of practising their modulation may represent a possible therapeutic approach useful for reducing the occurrence and development of numerous diseases associated with oxidative stress, such as for example inflammatory processes.

### Summary of the invention

The present invention is defined in the appended claims.

The object of the present description is to provide compositions with a high antioxidant capacity capable of counteracting the formation of free radicals through the synergistic effect exerted by the various components.

According to the invention, the aforesaid purpose is achieved thanks to the object specifically referred to in the following claims, which are to be understood as an integral part of the present description.

An embodiment of the present description provides a composition containing an active agent, said active agent comprising:
- at least one antioxidant compound selected from the group consisting of polyphenols and flavonoids,
- spermidine,
- at least one further component selected from trehalose and lentztrehalose.

In another embodiment, the present description provides a composition comprising an active agent, said active agent comprising spermidine, nicotinamide and at least one further component selected from trehalose and lentztrehalose.

Lentztrehalose can be selected from lentztrehalose A, B, C.

The Inventors of the present application have shown that, thanks to a synergistic action exerted by the different components of the active agent, the compositions as described are able to counteract oxidative stress and activate autophagy mechanisms.

In one or more embodiments, the compositions as described herein may also be used in medicine.

The compositions object of the present description can be used in the prevention and/or treatment of an oxidative stress disease selected in the group consisting of inflammation, heart failure, atherosclerotic disease, neurodegenerative diseases, neoplasms, hepatic steatosis, diabetes mellitus. In one or more embodiments, the composition can be used to treat an acute or chronic inflammatory state.

### Short description of the drawing

The invention will now be described, by way of example only, with reference to the attached figures, in which:

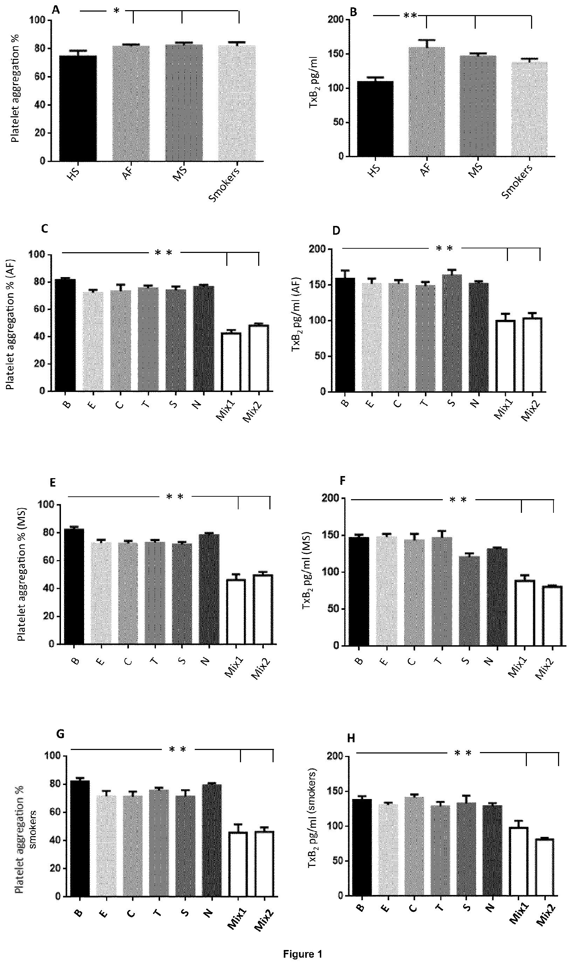

- **Figure 1** shows the results of an evaluation of the effect of the compositions object of the present description on platelet function. Evaluation of platelet aggregation (A) and TxB₂ production (B) in platelets of healthy subjects, patients with atrial fibrillation (AF; C and D), metabolic syndrome (MS; E and F) and smokers (G and H);

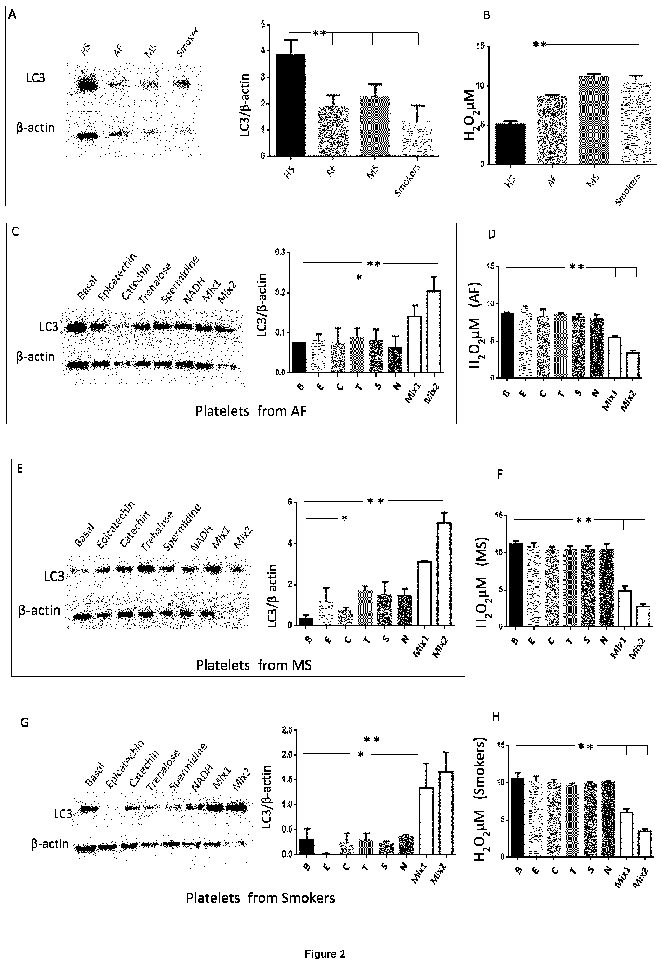

- **Figure 2** shows the results of an evaluation of the effect of the compositions object of the present description on autophagy and oxidative stress. Evaluation of autophagy (A) and H₂O₂ production (B) in platelets of healthy subjects, patients with atrial fibrillation (AF; C and D), metabolic syndrome (MS; E and F) and smokers (G and H);

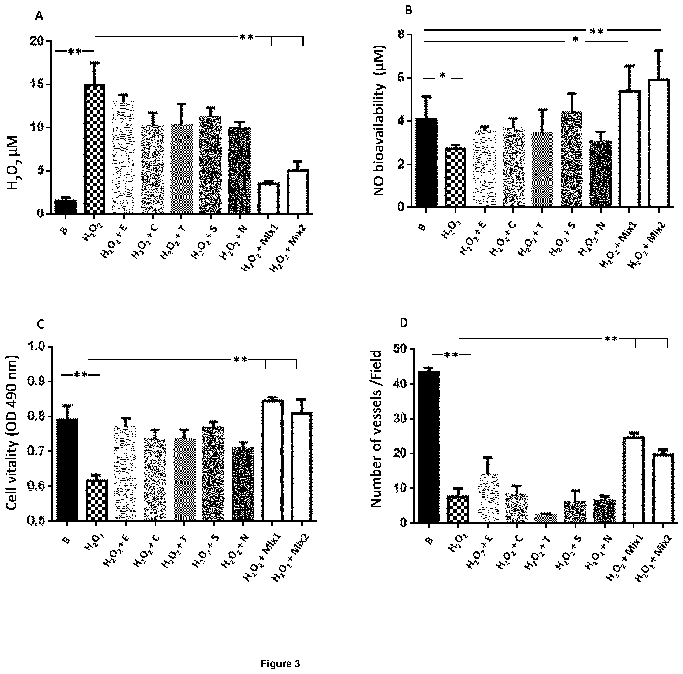

- **Figure 3** shows the results of an evaluation of the effect of the compositions object of the present description on oxidative stress, angiogenesis and vitality on endothelial cells (HUVEC). Effect of treatment of HUVEC cells with single components of the compositions and with the compositions object of the present description on the production of H₂O₂ (A) and on the bioavailability of nitric oxide (NO) (B), on cell viability (C) and angiogenesis (D).

### Detailed description of the invention

In the following description, several specific details are given to allow an exhaustive understanding of embodiments. The embodiments can be put into practice without one or more of the specific details, or with other processes, components, materials, etc. In other cases, well-known structures, materials or operations are not shown or described in detail to avoid obscuring aspects of the embodiments.

The reference throughout this description to "one embodiment" or "an embodiment" indicates that a particular aspect, structure or feature as described in relation to the embodiment is included in at least one embodiment. Thus, the appearances of the expressions "in one embodiment" or "in an embodiment" at various points throughout the present description do not all necessarily refer to the same embodiment. Furthermore, the particular aspects, structures or features can be combined in any suitable way in one or more embodiments.

The headings provided here are for convenience only.

An embodiment of the present description provides a composition comprising an active agent, said active agent comprising i) at least one antioxidant compound selected from the group consisting of polyphenols and flavonoids, ii) spermidine, iii) at least one further component selected from trehalose and lentztrehalose.

Lentztrehalose that can be used as a component of the active agent of the compositions as described can be lentztrehalose A, B or C.

A further embodiment provides a composition comprising an active agent, said active agent comprising spermidine, nicotinamide and a further component selected from trehalose and lentztrehalose. Also in this further embodiment, lentztrehalose can be lentztrehalose A, B or C.

The active principle of the compositions object of the present description can furthermore contain a trehalase inhibitor, preferably selected from trehazolin and validoxylamine A.

The compositions can be used in the prevention and/or treatment of an oxidative stress disease selected in the group consisting of inflammation, heart failure, atherosclerotic disease, neurodegenerative diseases, neoplasms, hepatic steatosis, diabetes mellitus.

The inflammatory state that can be prevented and/or treated by administering the compositions object of the present description is an acute or chronic inflammatory state.

With reference to this condition, there is a growing number of evidences suggesting that ROS may be implicated in systemic inflammation and endothelial dysfunction, that are factors that characterize pathological conditions such as cardiovascular diseases and tumours, (Carnevale et al., 2014, Lahera et al., 2007, Liou and Storz, 2010) but also conditions associated for example with smoking (Pittilo, 2000) or intense physical exercise (Ghisi, et al., 2010).

The analysis of autophagy, which is among the most important defense mechanisms against cellular damage induced by oxidative stress (Scherz-Shouval R et al., 2007) has allowed the inventors of the present application to demonstrate that the excessive production of ROS in the platelets of the patients examined was attributable to a decreased autophagic activity.

The compositions object of the present description have proved to be able - thanks to a synergistic effect exercised by the different components - to counteract the production of ROS, as illustrated below.

In fact, the present description demonstrates for the first time that *in vitro* administration of the compositions as described to platelets extracted from patients exerts an evident antioxidant and pro-autophagic effect.

The compositions object of the present description comprise, as active agent, trehalose (and/or lentztrehalose A, B or C) in combination with components with specific antioxidant activity.

Trehalose is a natural disaccharide composed of two glucose molecules bound by a α1-1-glycosidic bond. Trehalose is synthesized by lower organisms such as yeasts, insects, bacteria and plants but not by mammals. Trehalose performs multiple functions that distinguish it from other common disaccharides, including a protective action against various stressors, such as oxidative stress, temperature changes, accumulation of protein aggregates and dehydration. Furthermore, recent evidence has shown that trehalose could prevent inflammatory responses induced by endotoxic shock both *in vivo* and *in vitro.* Oral administration of this disaccharide has also been shown to reduce the development and progression of neurodegenerative disorders, hepatic steatosis, renal damage, insulin resistance, atherosclerosis, post-ischemic cardiac remodeling and pancreatitis (Schaeffer and Goedert, 2012; Zhang et al., 2014; DeBosch et al., 2016; Wang et al., 2017; Arai et al., 2010; Sciarretta et al., 2018) mainly through the stimulation of autophagy.

Lentztrehalose is a trehalose analogue whose structure is characterized by the presence of substituents on one of the glucose molecules; this structure determines a greater resistance to the degradation of the glycosyl bond by the trehalases. Lentztrehalose exhibits several biological activities that include the induction of autophagy (Zhang et al, 2015). Lentztrehalose may occur in different isoforms, such as isoform A, B, C.

Spermidine and at least one antioxidant compound selected in the group consisting of polyphenols and flavonoids can be contained in compositions according to embodiments of the present description in combination with trehalose and/or lentztrehalose A, B or C.

The group consisting of polyphenols and flavonoids can comprise both synthetic and derivative compounds, for example, from plants, fruits, natural drinks and oils.

In one or more embodiments, the group consisting of polyphenols and flavonoids can comprise the polyphenolic fraction of plants, fruits and oils.

In one or more embodiments, the group consisting of polyphenols and flavonoids can comprise epicatechin, catechin, curcumin, quercetin, resveratrol.

In one or more embodiments, the composition comprises an active agent containing i) at least one compound selected from the group consisting of polyphenols and flavonoids, preferably catechin and epicatechin, ii) spermidine, iii) at least one of trehalose and/or lentztrehalose.

Catechin and epicatechin are polyphenols capable of exerting an antioxidant effect; catechin, in particular, is a compound found in tea, cocoa and red wine.

Spermidine is a naturally occurring polyamine in some foods such as rice bran, soy, mushrooms and broccoli. Spermidine has been shown to prolong life span in yeasts, flies and worms in an autophagy-dependent manner.

Below, information are provided on the respective quantities of the components of the active agent of the compositions as described.

The at least one compound selected in the group consisting of polyphenols and flavonoids can be included in the compositions in an amount between 10% and 40% of the active principle.

In compositions comprising i) trehalose (and/or lentztrehalose A, B or C), ii) spermidine and iii) catechin and epicatechin as polyphenols, the amount of catechin can be between 10% and 40% (weight/weight), preferably equal to 25% (weight/weight) of the active agent.

The amount of spermidine can be between 0.025% and 5% (weight/weight), preferably equal to 0.15% (weight/weight) of the active agent.

The amount of epicatechin can be between 10% and 40% (weight/weight), preferably equal to 25% (weight/weight) of the active agent.

In one or more embodiments, the weight ratio between catechinin and epicatechin can be between 2:1 and 0.5:1, preferably equal to 1:1.

At least one component selected from trehalose and lentztrehalose (A, B or C) can be present in an amount between 15% and 79.5% (weight/weight), more preferably equal to 49.85% (weight/weight) of the active agent.

The weight ratio between the amount of trehalose (and/or lentztrehalose A, B or C) and the amount of catechin can be between 0.5:1 and 2:1.

The weight ratio between the amount of trehalose (and/or lentztrehalose A, B or C) and the amount of epicatechin can be between 0.5:1 and 2:1.

These dosages can allow a biologically active concentration to be reached in the circulation for each active component of the mixture.

In one or more embodiments, the active agent can consist of catechin, epicatechin, spermidine, and at least one of trehalose and/or lentztrehalose A, B or C.

The composition can further comprise at least one further component selected from vitamins, minerals, additives, flavouring substances. This additional component can be comprised in an amount by weight between 1% and 100% (weight/weight) with respect to the weight of the active agent.

For example, a composition according to embodiments can comprise the following components in the following quantities: epicatechin 250 mg, catechin 250 mg, trehalose and/or lentztrehalose (A, B or C) 498.5 mg, spermidine 1.5 mg, additives 100 mg.

A further embodiment of the composition as described is a composition which - in combination with a selected trehalose and lentztrehalose component - comprises spermidine and nicotinamide.

Lentztrehalose can be contained in the composition in the form of lentztrehalose A, B or C.

Nicotinamide is the precursor of coenzyme nicotinamide adenine dinucleotide (NAD+) and participates in cellular energy metabolism in the mitochondrial electron transport chain. It is also essential for the synthesis of nicotinamide adenine dinucleotide phosphate (NADP+), the basic molecule of fatty acid synthesis and cellular survival under conditions of oxidative stress. Experimental evidence has shown that nicotinamide can modify the redox balance *in vivo* and *in vitro*; a potential role as an antitumor agent has also been suggested, possibly in combination with other chemotherapeutic drugs based on *in vitro* studies on tumour cell models (Zhang et al., 2013; Audrito et al., 2011; Jung-Hynes et al., 2009; Dominguez-Gomez et al., 2015).

Compositions comprising, as active agent, trehalose and/or lentztrehalose A, B or C in combination with nicotinamide and spermidine can contain the different components in the quantities as indicated below.

Trehalose (and/or lentztrehalose A, B or C) can be present in an amount between 96.5% and 99.95% (weight/weight), preferably equal to 98.99% of the active agent.

Nicotinamide can be contained in an amount between 0.01% and 3% (weight/weight), preferably equal to 1% of the active agent.

The amount of spermidine can be between 0.001% and 1% (weight/weight), preferably equal to 0.01% of the active agent.

The weight ratio between the amount of nicotinamide and the amount of spermidine can be between 100:1 and 3:1.

In one or more embodiments, the active agent may consist of nicotinamide, spermidine and trehalose and/or lentztrehalose.

Also in this case, the composition can further comprise at least one further component selected from vitamins, minerals, additives, flavouring substances. This additional component can be comprised in an amount by weight between 1% and 100% (weight/weight) with respect to the weight of the active agent.

For example, a composition according to embodiments can comprise the following components in the following quantities: trehalose and/or lentztrehalose (A, B or C) 10000 mg, spermidine 1 mg, nicotinamide 100 mg, additives 500 mg.

In one or more embodiments, the compositions object of the present description can be administered as an oral nutritional supplement.

The compositions object of the present description can be administered, for example, in the form of tablets or in the form of granules to be dissolved in water or in drinks.

In one or more embodiments, the composition can be administered topically in the form of a gel, cream or ointment.

The Inventors of the present application have used different approaches to explore the antioxidant and autophagic activity of the compositions as described.

In particular, it has been shown that the compositions are able to under-regulate the production of platelet ROS, as shown below with reference to the quantitative evaluation of production of hydrogen peroxide (H₂O₂).

Furthermore, the reduction of oxidative stress was corroborated by the evident increase in autophagic activity demonstrated by a significant increase in the expression of LC3, one of the fundamental components of autophagosome formation during the autophagic process.

The excessive production of ROS in various diseases can have a potential effect on endothelial function (Taniyama and Griendling, 2003) and on apoptosis (Circu and Aw, 2010). Indeed, ROS can interfere with NO bioavailability, with the angiogenesis process favouring the apoptotic process through the induction of molecules such as caspases.

The present description provides evidence of a synergistic effect exerted by the components of the compositions according to the different embodiments.

As described in the following sections, the in vitro treatment with the compositions is associated with an increase in cell viability in HUVEC cells, as demonstrated by the cell proliferation assay (MTS, 3-(4,5-dimethylthiazol-2-yl)-5-(3-carboxymethoxyphenyl)-2-(4-sulfophenyl)-2H-tetrazolium, inner salt).

Furthermore, the improved viability was corroborated by i) increased bioavailability of NO, which plays an important role in protecting against the onset and progression of cardiovascular disease, and ii) increased angiogenesis as demonstrated by an increase in vessel formation capacity *in vitro.*

The Inventors of the present application have conducted experimental tests verifying the effect of the components alone and in combination. The results of the experimental tests have shown a surprising synergistic effect exerted by the different components of the active agent on oxidative stress, autophagy, platelet and endothelial function.

On the contrary, the in vitro administration of the individual components of each composition has not been shown to be effective in contrasting the production of H₂O₂ and platelet aggregation. Furthermore, the administration of the individual components did not result in a significant increase in the bioavailability of NO, autophagy, cell viability and angiogenesis.

The present description provides the first evidence that the effect of trehalose can be enhanced when in combination with polyphenols and antioxidant molecules as described herein, effectively counteracting oxidative stress and endothelial function through an autophagy-mediated mechanism.

### MATERIALS AND METHODS

### Study and recruitment population

Five healthy subjects (3 males and 2 females, age 61.2 ± 8.3), 5 patients with atrial fibrillation (FA) (3 males and 2 females, age 61.6 ± 10.1), 5 patients with metabolic syndrome (MS) (3 males and 2, age 68.4 ± 6.8) and 5 smokers (3 males and 2 females, age 30.8 ± 6.7) gave informed consent to participate in the study that was conducted between March and May 2018 at the Policlinico Umberto I (Rome).

### Platelet preparation

The subjects' whole blood was collected in tubes containing 3.2% sodium citrate as an anti-coagulant (sodium citrate/whole blood ratio, 1:9). To obtain platelet-rich plasma (PRP), blood samples were centrifuged for 15 minutes at 180 g at room temperature (about 21°C). Two thirds of the PRP (75%) were transferred to a new plastic tube using a transfer pipette without disturbing the buffy coat layer, in order to avoid contamination. Platelet poor plasma (PPP) was prepared by centrifuging the remaining sample at 300 g for 10 minutes at room temperature. The platelet function test was performed in three hours.

### Preparation of the composition comprising trehalose, catechin, epicatechin, spermidine (Mix1)

The various components were purchased from Sigma Aldrich (cod T9631 for trehalose; cod C0567 for catechin; cod E4018 for epicatechin; cod S2626 for spermidine). Mix 1 was prepared from the stock solutions of each component which was diluted in H₂O (trehalose and spermidine) and in dimethyl sulphate (DMSO; epicatechin and catechin) to obtain a concentration of 10 µM for trehalose, 10 µM for catechin, 10 µM for epicatechin and 5 µM for spermidine.

### Preparation of the composition comprising trehalose, spermidine, nicotinamide (Mix2)

The various components were purchased from Sigma Aldrich (cod T9631 for trehalose; cod S2626 for spermidine; cod N3376 for nicotinamide). Mix 2 was prepared from the stock solutions of each component that was diluted in water to obtain a concentration of 10 µM for trehalose, 5 µM for spermidine and 5 µM for nicotinamide.

### Platelet aggregation

Platelet aggregation was performed on PRP and was monitored using the ChronoLog Model 700 light transmission aggregometer.

Before activation with collagen, several samples were pre-incubated (20 minutes at 37°C) with compositions containing the following components:
- Composition 1: epicatechin (E, 10 µM)
- Composition 2: catechin (C, 10 µM)
- Composition 3: trehalose (T, 10 µM)
- Composition 4: spermidine (S, 5 µM)
- Composition 5: nicotinamide (N, 5 µM).

In order to evaluate the possible synergistic effect exercised by the combination of the specific components, further samples were pre-incubated (20 minutes at 37°C) with:
i) a composition comprising epicatechin (10 µM), catechin (10 µM), trehalose (10 µM), spermidine (5 µM) ("Mix 1");
ii) a composition comprising trehalose (10 µM), spermidine (5 µM), nicotinamide (5 µM) ("Mix 2").

After incubation, the samples are activated with collagen (Mascia Brunelli, 2 µg/ml) for 10 minutes at 37°C.

After stimulation, the ACD solution (Citric Acid Dextrose) was added to the samples and then the samples were centrifuged for 3 minutes at 3000 g. The supernatants were stored at -80°C for the analysis of soluble biomarkers and the pellets were stored at -80°C for autophagy analysis. The intra-assay and inter-assay variation coefficients for the aggregation test are 5.7% and 6.8% respectively.

### Evaluation of H₂O₂ production

H₂O₂ production was evaluated by a colorimetric assay (Arbor Assay) and expressed in µM. The intra-assay and inter-assay variation coefficients were 2.1% and 3.7% respectively.

### Evaluation of autophagy by Western blot analysis

The proteins (30 µg/well), whose concentration was estimated by the Bradford method, were solubilized in 2X Leammli buffer containing 20% of 2-mercaptoethanol and then boiled for 5 minutes at 95°C. The proteins were separated on 15% gel and then transferred onto nitrocellulose membranes (Trans-Blot Turbo Mini Nitrocellulose, Bio-Rad). Subsequently, the membranes were blocked with 5% (w/v) of milk dissolved in Tris solution 1X and 0.1% Tween. The membranes were then incubated with anti-LC3 antibody (MBL) and anti-α-actin monoclonal antibody and incubated at 4°C overnight. Finally, the membranes were incubated with the secondary antibody (Santa Cruz Biotechnology, 1:5000) and the immune complexes visualized with a chemiluminescent substrate. The densitometric analysis of the bands was performed using the software Image J.

### HUVEC cell treatment

HUVEC cells (Lonza, Cat. N.CC-C2517A) were cultured in complete EGM-2 medium (Lonza Cat. N. CC-3162) and were diluted to a density of 2500 cells/cm². The experiments were conducted on three different HUVEC cell lots. Cell cultures were tested between steps 3-6.

### Angiogenesis

50 µl of extracellular matrix (Matrigel) were deposited in a 96-well plate which was subsequently incubated at 37°C for 60 minutes. An equal number of cells (3x10³ cells/cm²) were initially treated for 2 h with culture medium in the absence of serum and subsequently treated with culture medium (without serum), H₂O₂ (800 µM) with or without epicatechin (10 µM) or catechin (10 µM) or trehalose (10 µM) or spermidine (5 µM) or nicotinamide (5 µM) or composition "Mix1" or composition "Mix2" for 3 h. After 3 h, the cells were detached and plated at a concentration of 1X10³ cells/cm² in each well of the 96-well plate containing the Matrigel and incubated for 8 h. The presence of capillary-like structures was evaluated under a phase-contrast microscope. Vessel quantification was expressed as an average of the number of vessels in 5 random fields.

### Cell viability test (MTS)

HUVEC cells were plated in triplicate at the concentration of 3x10³/well in a 96 plate. They were allowed to adhere for 24 h after which the treatment was performed. The cells were treated for 2 h with medium in the absence of serum. Subsequently the cells were treated with 100 µl of medium (without serum) containing the various components for 3 h. At the end of 3 h, 20 µl of MTS were added to the medium and the plate was incubated at 37°C for 2h. At the end of the two hours the wavelength at 490 nm was measured with a microplate reader (Tecan Magellan).

### Determination of NO bioavailability

A colorimetric assay kit (Tema Ricerca, Italy) was used to determine the NO metabolites, i.e. nitrites and nitrates (NOx) in 100 µl of sample maintained under agitation conditions for 10 minutes at 37°C. The intra-assay and inter-assay variation coefficients are 2.9% and 1.7% respectively.

### Statistical procedures

Continuous variables are reported as means ± standard deviation. Comparisons between the groups were made by Student's t-test and were replicated as appropriate with non-parametric tests (Kolmogorov-Smirnov test (z) in the case of non-homogeneous variances as verified by the Levene test). The results were expressed as mean ± SD. A value p<0.05 was considered statistically significant. All analyses were performed using GraphPad Prism 7 (GraphPad Software La Jolla, CA 92037 USA).

### RESULTS

### Effect of compositions on platelet function, oxidative stress, autophagy

Compared to platelets isolated from healthy subjects, platelets isolated from patients with atrial fibrillation (AF), patients with metabolic syndrome (MS) and smokers are more reactive as demonstrated by the increase:
- of the aggregation in response to collagen (74.6 ± 4.1% vs 81.6 ± 1.5% AF, p = 0.03; 74.6 ± 4.1% vs 82.33 ± 1.6 % MS, p = 0.02; 74.6 ± 4.1% vs 82.0 ± 2.6% smokers, p = 0.03) (Figure 1A) and
- increased production of Thromboxane B2 (129.7 ± 6.8 pg/ml vs 159.0 ± 11.8 pg/ml] AF, p = 0.002; 129.7 ± 6.8 pg/ml vs 146,7 ± 4.7 pg/ml MS p = 0.001, 129.7 ± 6.8 pg/ml vs 138.0 ± 5.5 pg/ml smokers, p = 0.008) (Figure 1B).

On other samples of the same groups of subjects, the degree of autophagy was also evaluated by quantitative analysis of the protein associated with LC3 microtubules, which is an autophagosome marker.

The analysis performed by western blot showed that the expression of LC3 protein is significantly reduced in the platelets of patients compared to that of healthy subjects (1.95 ± 0.07 AU vs 0.075 ± 0.07 AU AF, p = 0.0005; 1.95 ± 0.07 AU vs 0.3 ± 0.14 AU MS, p = 0.0008, 1.95 ± 0.07 AU vs 0.29 ± 0.24 AU smokers, p = 0,0007) (Figure 2A).

This evidence is also associated with an increase in oxidative stress, as demonstrated by the significant increase in H₂O₂ production (5.1 ± 0.4 µM vs 8.6 ± 1.5 µM AF, p<0.001; 5.1 ± 0.4 µM vs 11.17 ± 1.4 µM MS, p<0.001; 5.1 ± 0.4 µM vs 9.8 ± 1.0 µM smokers, p<0.001) (Figure 2B).

The synergistic effect exerted by the components of the active agent of the compositions as described was demonstrated following platelet treatment with the components individually and in combination administered.

The results show that treatment of platelets with individually administered components does not induce significant changes:
- in platelet aggregation (Figure 1C, E, G),
- in the production of thromboxane (Figure 1D, F, H),
- in the autophagic process (Figure 2C, E, G),
- in oxidative stress (Figure 2D, F, H).

On the contrary, the administration of the two compositions ("Mix1") and ("Mix2") significantly reduces:
- platelet aggregation (Figure 1C, E, G),
- thromboxane levels (Figure 1D, F, H),
- oxidative stress (Figure 2D, F, H),
and determines an increase in autophagic processes (Figure 2C, E and G).

The compositions object of the present description also protect the HUVEC cells from the oxidative stress induced by H₂O₂, favour angiogenesis and cellular vitality.

Oxidative stress and NO bioavailability were evaluated following stimulation of cells with H₂O₂.

In particular, treatment with the individual components of the first composition ("MIX1") and the second composition ("Mix2") results in a non-significant reduction in H₂O₂ production and a non-significant increase in the NO bioavailability (Figure 3A and B).

Similarly, there are no significant changes in cell viability (Figure 3C) and in the number of vessels (Figure 3D).

In contrast, pre-treatment with the compositions, as described, of HUVEC cells stimulated with H₂O₂ induces a significant reduction in H₂O₂ production (Figure 3A), an increase in the NO bioavailability (Figure 3B), an increase in cell viability (Figure 3C) and the number of blood vessels (Figure 3D).

The compositions as described here therefore, thanks to the synergic effect exerted by the various components, are able to significantly counteract the damage exerted by oxidative stress and enhance endothelial function through a mechanism mediated by autophagy.

### REFERENCES

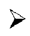 Finkel, T. e N. J. Holbrook (2000). "Oxidants, oxidative stress and the biology of ageing". Nature 408(6809) :239-247.
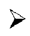 Ganceviciene, R., A. I. Liakou, A. Theodoridis, E. Makrantonaki e C. C. Zouboulis (2012). "Skin anti-aging strategies". Dermatoendocrinol 4(3) : 308-319.
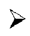 Guo, H., Huang, K., Zhang, X., Zhang, W., Guan, L., Kuang, D., Deng, Q., Deng, H., Zhang, X., He, M., Christiani, D., Wu, T. (2014). "Women are more susceptible than men to oxidative stress and chromosome damage caused by polycyclic aromatic hydrocarbons exposure". Environ Mol Mutagen. [Epub ahead of print]
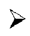 Hao, L., Huang, H., Gao, J., Marshall, C., Chen, Y., Xiao, M. (2014). "The influence of gender, age and treatment time on brain oxidative stress and memory impairment induced by d-galactose in mice". Neurosci Lett 571C:45-49.
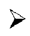 Kohen, R. (1999). "Skin antioxidants: their role in aging and in oxidative stress--new approaches for their evaluation". Biomed Pharmacother 53(4):181-192.
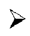 Kozina, L. S., I. V. Borzova, V. A. Arutiunov e G. A. Ryzhak (2012). "The role of oxidative stress in skin aging". Adv Gerontol 25(2):217-222.
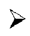 Kunwar, A. e K.I. Pridashini (2011). "Free radicals, oxidative stress and importance of antioxidants on human health". J Med Applied Sci 1(2):3-60.
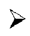 Levine, R. L. e. R. Stadtman (2001). "Oxidative modification of proteins during aging". Exp Gerontol 36(9): 1495-1502.
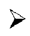 Sohal, R.A. e R. Weindruch (1996). "Oxidative stress, caloric restriction, and aging". Science 273 (5271) : 59-63.
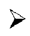 Turrens, J.F. e J.M. McCord (1990). "Free Radicals, Lipoproteins, and Membrane Lipids". Paulet, A.C.; Douste-Blazy, L.; Paoletti, R., editors, Plenum; New York: 203-212.
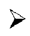 Zhang, M., Wada, S., Amemiya, F, Watanabe, T., Shibasaki, M (2015). "Synthesis and Determination of Absolute Configuration of Lentztrehalose A". Chem Pharm Bull 63(11) :961-966.

## Claims

1. Antioxidant composition containing an active agent, said active agent comprising nicotinamide, spermidine and at least one further component selected from trehalose and lentztrehalose.

2. Antioxidant composition according to claim 1, wherein the amount of nicotinamide is comprised between 0.01% and 3% (weight/weight) of the active agent.

3. Antioxidant composition according to claim 1 or claim 2, wherein the amount of spermidine is comprised between 0.001% and 1% (weight/weight) of the active agent weight.

4. Antioxidant composition according to any one of the previous claims, wherein the amount of trehalose and/or lentztrehalose is comprised between 96.5% and 99.95% (weight/weight) of the active agent.

5. Antioxidant composition according to any one of the previous claim, wherein the weight ratio between the nicotinamide amount and the spermidine amount is between 100:1 and 3:1.

6. Antioxidant composition according to any one of the previous claim, wherein said active principle further comprises at least one trehalase inhibitor, preferably selected from trehazolin and validoxylamine A.

7. Antioxidant composition according to any one of the previous claim, wherein the composition comprises at least one further component selected in the group consisting of water, vitamins, minerals, additives, flavouring substances.

8. Antioxidant composition according to claim 7, wherein said at least one further component is comprised in a weight amount comprised between 1% and 100% (weight/weight) with respect to the weight of the active agent.

9. Antioxidant composition according to any one of the previous claim, wherein said composition is administered in the form of tablets, granules to be dissolved in water or in beverages and/or as a preformed aqueous solution.

10. Antioxidant composition according to any one of the previous claim, wherein the composition is administered as an oral nutritional supplement.

11. Antioxidant composition according to any one of the previous claim, for use in medicine.

12. Antioxidant composition according to any one of the previous claim, for use in the treatment of an oxidative stress disease selected in the group consisting of inflammation, heart failure, atherosclerotic disease, neurodegenerative diseases, neoplasms, hepatic steatosis, diabetes mellitus.

13. Antioxidant composition for use according to claim 12, wherein the inflammation comprises an acute or chronic inflammatory state.

## Patentansprüche

1. Antioxidanszusammensetzung, die einen Wirkstoff enthält, wobei der Wirkstoff Nicotinamid, Spermidin und mindestens eine weitere Komponente, die gewählt ist aus Trehalose und Lentztrehalose, umfasst.

2. Antioxidanszusammensetzung nach Anspruch 1, wobei die Menge an Nicotinamid zwischen 0,01 % und 3 % (w/w) des Wirkstoffs liegt.

3. Antioxidanszusammensetzung nach Anspruch 1 oder 2, wobei die Menge an Spermidin zwischen 0,001 % und 1 % (w/w) des Wirkstoffs liegt.

4. Antioxidanszusammensetzung nach einem der vorhergehenden Ansprüche, wobei die Menge an Trehalose und/oder Lentztrehalose zwischen 96,5 % und 99,95 % (w/w) des Wirkstoffs liegt.

5. Antioxidanszusammensetzung nach einem der vorhergehenden Ansprüche, wobei das Massenverhältnis der Menge an Nicotinamid und der Menge an Spermidin zwischen 100:1 und 3:1 liegt.

6. Antioxidanszusammensetzung nach einem der vorhergehenden Ansprüche, wobei der Wirkstoff ferner mindestens einen Trehalasehemmer umfasst, vorzugsweise Trehazolin oder Validoxylamin A.

7. Antioxidanszusammensetzung nach einem der vorhergehenden Ansprüche, wobei die Zusammensetzung mindestens eine weitere Komponente umfasst, ausgewählt aus der Gruppe, bestehend aus Wasser, Vitaminen, Mineralien, Additiven, Aromastoffen.

8. Antioxidanszusammensetzung nach Anspruch 7, wobei die mindestens eine weitere Komponente in einem Gewichtsanteil von zwischen 1 % und 100 % (w/w) bezogen auf das Gewicht des Wirkstoffs umfasst ist.

9. Antioxidanszusammensetzung nach einem der vorhergehenden Ansprüche, wobei die Zusammensetzung in Form von Tabletten, Granulat zum Lösen in Wasser oder in Getränken, und/oder als vorgefertigte wässrige Lösung verabreicht wird.

10. Antioxidanszusammensetzung nach einem der vorhergehenden Ansprüche, wobei die Zusammensetzung als orales Nahrungsergänzungsmittel verabreicht wird.

11. Antioxidanszusammensetzung nach einem der vorhergehenden Ansprüche zur Verwendung in Medikamenten.

12. Antioxidanszusammensetzung nach einem der vorhergehenden Ansprüche zur Verwendung bei der Behandlung einer oxidativen Stresserkrankung, ausgewählt aus der Gruppe bestehend aus Entzündung, Herzversagen, atherosklerotischer Erkrankung, neurodegenerativen Erkrankungen, Neoplasmen, hepatischer Steatose, Diabetes mellitus besteht.

13. Antioxidanszusammensetzung zur Verwendung nach Anspruch 12, wobei die Entzündung einen akuten oder chronischen Entzündungszustand umfasst.

## Revendications

1. Composition antioxydante contenant un principe actif, ledit principe actif comprenant du nicotinamide, de la spermidine et au moins un autre composant choisi parmi le tréhalose et le tréhalose de Lentz.

2. Composition antioxydante selon la revendication 1, dans laquelle la quantité de nicotinamide est comprise entre 0,01 % et 3 % (poids/poids) du principe actif.

3. Composition antioxydante selon la revendication 1 ou la revendication 2, dans laquelle la quantité de spermidine est comprise entre 0,001 % et 1 % (poids/poids) du poids du principe actif.

4. Composition antioxydante selon l'une quelconque des revendications précédentes, dans laquelle la quantité de tréhalose et/ou de tréhalose de Lentz est comprise entre 96,5 % et 99,95 % (poids/poids) du principe actif.

5. Composition antioxydante selon l'une quelconque des revendications précédentes, dans laquelle le rapport en poids entre la quantité de nicotinamide et la quantité de spermidine est compris entre 100:1 et 3:1.

6. Composition antioxydante selon l'une quelconque des revendications précédentes, dans laquelle ledit principe actif comprend en outre au moins un inhibiteur de tréhalase, de préférence choisi parmi la tréhazoline et la validoxylamine A.

7. Composition antioxydante selon l'une quelconque des revendications précédentes, laquelle composition comprend au moins un autre composant choisi dans le groupe constitué par l'eau, les vitamines, les minéraux, les additifs, et les substances aromatisantes.

8. Composition antioxydante selon la revendication 7, dans laquelle ledit au moins un autre composant est compris en une quantité en poids comprise entre 1 % et 100 % (poids/poids) par rapport au poids du principe actif.

9. Composition antioxydante selon l'une quelconque des revendications précédentes, laquelle composition est administrée sous la forme de comprimés, de granules à dissoudre dans de l'eau ou dans des boissons, et/ou d'une solution aqueuse préformée.

10. Composition antioxydante selon l'une quelconque des revendications précédentes, laquelle composition est administrée sous la forme d'un supplément nutritionnel à usage oral.

11. Composition antioxydante selon l'une quelconque des revendications précédentes, pour une utilisation en médecine.

12. Composition antioxydante selon l'une quelconque des revendications précédentes, pour une utilisation dans le traitement d'une maladie de stress oxydatif choisie dans le groupe constitué par une inflammation, une insuffisance cardiaque, une maladie athérosclérotique, une maladie neurodégénérative, un néoplasme, une stéatose hépatique, un diabète sucré.

13. Composition antioxydante pour une utilisation selon la revendication 12, dans laquelle l'inflammation comprend un état inflammatoire aigu ou chronique.
